# EUROPEAN PATENT APPLICATION

(11) **EP 3 321 376 A1**
(43) Date of publication of application: **16.05.2018**
(21) Application number: 16382527.6
(22) Date of filing: 11.11.2016
(51) Int. Cl.: C12Q 1/68

(54) **ELECTROCHEMICAL DNA DETECTION**

(71) Applicant: Genomica S.A.U., 28033 Madrid (ES)
(72) Inventor: Villahermosa Jaen, María Luisa, 28033 Madrid (ES); Calvo Macarro, Francisco Javier, 28033 Madrid (ES)
(74) Representative: ZBM Patents - Zea, Barlocci & Markvardsen

(57) **Abstract**

Electrochemical DNA detection methods are disclosed comprising the steps of: i) Amplifying a target DNA with a pair of amplification primers, wherein at least one of the primers is chemically linked to a label through its 5' end; ii) Denaturing the amplified product to yield two molecules of single stranded DNA (ssDNA) of which at least one is 5'-labeled; iii) Hybridizing a 5'-labeled ssDNA molecule of the amplified product with a complementary oligonucleotide capture probe which is attached through its 3' end to an electrode; and iv) Determining the presence of the label associated with the hybridization product; wherein the distance between the 5'-end and the first nucleotide of the hybridization region within the ssDNA molecule that hybridises to the probe is comprised from 0 to 300 nucleotides. Cartridge devices, operator devices, methods of controlling an operator device, and computer programs implementing said methods are also disclosed, which are suitable for performing such electrochemical DNA detection methods.

## Description

### Technical field

The present disclosure relates to electrochemical DNA detection methods. Further, the disclosure relates to cartridge devices, operator devices, methods of controlling an operator device, and computer programs implementing such methods, said devices, methods and programs being suitable for performing the above electrochemical DNA detection methods.

### Background Art

Recently, microarray technology has been developed to facilitate large scale nucleic acid analysis enabling the simultaneous analysis of thousands of DNA sequences (see for example U.S. Patent No. 5,445,93 or WO2007017699). The term microarray is meant to indicate analysis of many small spots. Is usually performed on a solid support and may also be performed on small scale. The microarray technology has been successfully applied to several fields, for example in HPV diagnosis (see Patent Publications WO0168915 and No. CA2484681).

However, there is still a drawback with the use of microarray technology as expensive equipment and laborious handling are required. For example the CombiMatrix CustomArray® microarray and ElectraSense microarray are complementary metal oxide semiconductor (CMOS) chips with 12,544 electrodes that can be addressed individually or in user-defined groups (see US 20110281766A1). These arrays are available commercially as custom DNA chips with different nucleic acid probe sequences produced at each electrode using sequential electrochemical reactions to add phosphoramidites. Hybridization to probes can be detected using cyanine (Cy) dyes and fluorescent scanners or, alternatively, using horseradish peroxidase (HRP) and enzyme-enhanced electrochemical detection (ECD) on CombiMatrix's microarray readers. The CombiMatrix microarray supports *in situ* electrochemical synthesis of user-defined DNA probes. CombiMatrix microarrays were initially developed as highly multiplexed platforms for electrochemistry. The original complementary metal oxide (CMOS) microarray had 1,000 platinum (Pt) electrodes (1 K microarray), and it was used to develop the in situ electrochemical synthesis of different DNA probes on individual electrodes. Hybridization to these probes was detected using enzyme-enhanced electrochemical detection (ECD) (Dill, K.; Montgomery, D. D.; Ghindilis, A. L.; Schwarzkopf, K. R. Immunoassays and sequence-specific DNA detection on a microchip using enzyme amplified electrochemical detection. J Biochem. Biophys. Methods 2004, 59, 181-187). A 12K CustomArray® microarray is commercially available as a custom gene chip that has been used for a variety of genomic assays (e.g., genotyping, gene expression, SNP analysis, etc.).

In the field of point-of-care diagnosis more than portable equipment is needed, it is necessary to have a robust, sensitive, easy-handling system but without compromising the reliability of the diagnosis.

### Summary of Invention

The present invention provides improved methods for electrochemical detection of DNA. The inventors have developed a robust, sensitive and reliable method for electrochemical DNA detection comprising amplification, hybridization with a probe and electrochemical detection. The inventors have surprisingly found effect that wherein the probes are attached through its 3' end to the electrode and the distance within the molecule to be hybridized to the probe between the 5'-end and the first nucleotide of the hybridization region is comprised from 0 to 300 nucleotides the diagnosis is better and more reliable.

In a first aspect, the invention refers to an electrochemical DNA detection method comprising the steps of:
i) Amplifying a target DNA with a pair of amplification primers, wherein at least one of the primers is chemically linked to a label through its 5' end;
ii) Denaturing the amplified product to yield two molecules of single stranded DNA (ssDNA) of which at least one is 5'-labeled;
iii) Hybridizing a 5'-labeled ssDNA molecule of the amplified product with a complementary oligonucleotide capture probe which is attached through its 3' end to an electrode; and
iv) determining the presence of the label associated with the hybridization product, wherein the distance between the 5'-end and the first nucleotide of the hybridization region within the ssDNA molecule is comprised of from 0 to 300 nucleotides.

The inventors have surprisingly found that the present method provides a significant increase in signal detection, resulting in an improved sensitivity and specificity in DNA detection when compared with prior art methods.

The present invention additionally provides improved cartridge devices, operator devices, methods of controlling an operator device, and computer programs implementing such methods, said devices, methods and programs being suitable for performing the above electrochemical DNA detection methods.

In order to carry out the method of the invention, adequate means are required. Thus a second aspect of the invention refers to use of means for electrochemical DNA detection as defined above, wherein the means comprise:
i) A pair of primers to amplify the DNA target, wherein at least one of the primers is chemically linked to a label through its 5' end;
ii) An oligonucleotide capture probe complementary to the DNA target sequence, wherein the oligonucleotide capture probe is attached through its 3' end to an electrode; and
iii) means to detect the label;

Wherein when in use the distance between the 5'-end and the first nucleotide of the hybridization region within the ssDNA molecule that hybridises to the probe is comprised from 0 to 300 nucleotides.

The means to carry out the method of the invention may be included in a cartridge device. A third aspect of the invention thus refers to a cartridge device for electrochemical detection including a microfluidic circuit comprising:
a microfluidic amplification chamber operable to cause amplification of a target DNA with a pair of amplification primers, wherein at least one of the primers is chemically linked to a label through its 5' end, and to cause denaturation of the amplified product to yield two molecules of single stranded DNA (ssDNA) of which at least one is 5'-labeled; and
a microfluidic sensor chamber comprising one or more electrodes and one or more complementary oligonucleotide capture probes attached through their 3' end to the one or more electrodes, and being operable to cause hybridization of the at least one 5'-labeled ssDNA molecule of the amplified product with the one or more complementary oligonucleotide capture probes, the distance upon hybridization between the 5'-end and the first nucleotide of the hybridization region within the ssDNA molecule that hybridises to the probe being of from 0 to 300 nucleotides; wherein
the microfluidic sensor chamber is further operable to cause determination of the presence of the label associated with the hybridization product.

All or part of the means (reagents, electrodes, capture probes, primers, etc.) required to detect one or more particular DNA targets may be preloaded onto the cartridge. For example, all or part of the fluid/liquid means may be preloaded in corresponding containers, blisters, etc.

The microfluidic sensor chamber may comprise an array of electrodes (i.e. more than one electrode) with different complementary oligonucleotide capture probes attached thereto. Different configurations are possible in this respect. For example, different electrodes may have different probes and/or the same probe attached thereto, and/or the same electrode may have more than one probe attached thereto, etc. depending on the detection to be performed.

In some examples, the cartridge device may further comprise a sample inlet for receiving the target DNA.

According to some implementations, the cartridge device may further comprise a container containing a hybridization buffer and in fluid communication with the microfluidic circuit, so that the hybridization buffer is transferrable to the microfluidic sensor chamber.

The cartridge may be sold ready to be used in combination with an operator device such that the user needs only load the sample containing the target DNA onto the cartridge and associate (e.g. insert) it in the operator device to directly obtain a result indicating the presence or absence and even the quantity of the target DNA in the sample. An overall system may comprise a consumable cartridge device with integrated (array of) electrodes and respective operator device (or instrument) to operate and read-out the cartridge device. Such devices are often generally referred to as lab-on-a-chip.

Another aspect of the invention refers to a method of controlling an operator device for operating a cartridge device as defined above, which is associable (attachable, connectable, etc.) to the operator device.

The "control" method (of controlling the operator device) comprises providing, by a controller (of the operator device), control signal to a first heater unit (of the operator device) for operating the microfluidic amplification chamber (of the cartridge device) by providing first heating cycles to the microfluidic amplification chamber, so that amplification and denaturation occur in the microfluidic amplification chamber.

The control method further comprises providing, by the controller, control signal to a second heater unit (of the operator device) for operating the microfluidic sensor chamber (of the cartridge device) by providing second heating cycles to the microfluidic sensor chamber, so that hybridization occurs in the microfluidic sensor chamber.

The control method still further comprises providing, by the controller, control signal to a detection unit (of the operator device) for operating the microfluidic sensor chamber so that determination of the presence of the label associated with the hybridization product occurs.

In a further aspect, the invention provides an operator device for operating a cartridge device which is associable to the operator device, wherein the operator device comprises a first heater unit, a second heater unit, a detection unit, and a controller. The controller may be implemented by computing means, electronic means or a combination thereof, as described in more detail in other parts of the disclosure.

In a still further aspect, the invention also refers to a computer program product comprising program instructions for causing a controller (of an operator device) to perform a method as defined above of controlling an operator device for operating a cartridge device.

The present DNA detection method, means to carry out such detection, cartridge device, operator device, and computer program are not restricted to any particular DNA. Any target DNA can be detected as long as the conditions disclosed in the first aspect of the invention are present. Particular relevance is given to the primer design which determines that the distance between the 5'-end and the first nucleotide of the hybridization region within the ssDNA molecule to be detected is of from 0 to 300 nucleotides, and also to the orientation of the capture probe, which is attached to the electrode through its 3'-end.

Other relevant features are related to the method configuration (i.e. 5'-labelled primer and capture probe attached to the electrode through its 3' end). As an example the inventors have detected human papilloma virus (HPV) in human samples and carrying out the method on a cartridge device (in combination with corresponding operator device) according to the invention. The employed cartridge device and the conditions to detect human HPV DNA are described in the examples below. The obtained results show that the intensity of the detection signal is surprisingly increased when the configuration of the invention is used.

While being useful for detecting any type of DNA target, the method and devices of the invention may be used in the diagnosis of a wide variety of diseases, and also for the detection of a wide variety of microorganisms, preferably pathogenic microorganisms. Thus the method and devices of the present invention find application in the fields of medicine, in particular oncology and prenatal testing, veterinary, microbiology, food science and environmental science. Further, in as far as the sample to be analyzed comprises nucleic acids, characterization of such a sample may be performed in the context of the proposed detection methods and devices.

These and other advantages and features of the invention will become apparent in view of the detailed description and drawings.

### Brief description of the drawings

Non-limiting examples of the present disclosure will be described in the following, with reference to the appended drawings, in which:

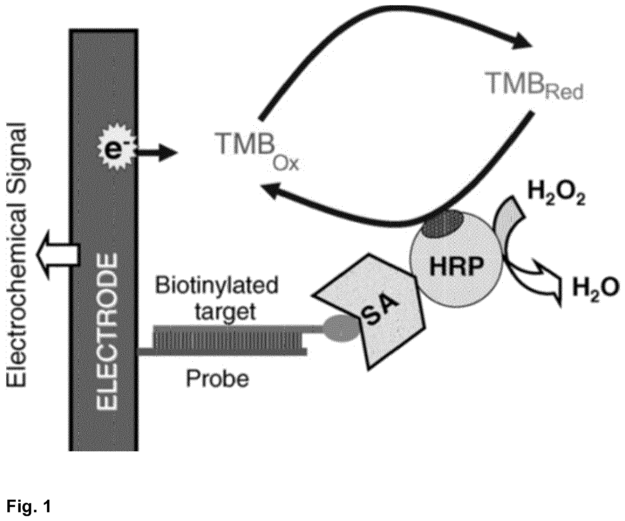

Figure 1 is a schematic representation of the electrochemical detection system of the closest prior art CombiMatrix. TMB, 3,3',5,5'-tetramethylbenzidine; Red, reduced; Ox, oxidated; SA, streptavidin; HRP, horse radish peroxidase.
Figure 2 is a schematic representation of the present invention. The details provided correspond to particular conditions (distances and DNA modifications) of Example 1 of the invention. S 8.5, probe with thiol in its 5' end; S 8.3, probe with thiol in its 3' end; S 7.5, probe with thiol in its 5' end; S 7.3, probe with thiol in its 3' end; •, biotin; ■, thiol
Figure 3 is a schematic representation of displays of the detection signals (in nA) obtained with the different probes of the invention. S 8.5, probe with thiol in its 5' end; S 8.3, probe with thiol in its 3' end; S 7.5, probe with thiol in its 5' end; S 7.3, probe with thiol in its 3' end.
Figure 4 is a schematic representation of the microscopic events that may be taking place within the array with the probes of the invention. S 8.5, probe with thiol in its 5' end; S 8.3, probe with thiol in its 3' end; S 7.5, probe with thiol in its 5' end; S 7.3, probe with thiol in its 3' end; Dot, biotin.
Figure 5a is a schematic representation of a cartridge device suitable for performing DNA detection methods similar to the ones illustrated by other figures, according to examples;
Figure 5b is a schematic representation of an electrode microarray suitable for cartridge devices similar to the one illustrated by Figure 5a, according to examples;
Figure 6 schematically illustrates an operator device suitable for operating cartridge devices similar to the ones illustrated by Figure 5a, according to examples;
Figure 7 is a flowchart schematically illustrating a method of controlling an operator device similar to the ones illustrated by Figure 6, according to examples.
Figure 8 represents the detection signals (in nA) of the electrode array for HPV detection. "br" is biotine marker; "ic", internal control of amplification; "ec",endogenous control; A, HPV 39; B, HPV 45; C, HPV 16; D, HPV 18; E, HPV 31; F, HPV33; G, HPV 35; H, HPV 51; I, HPV 52; J, HPV 56; K, HPV 58; L, HPV 59; M, HPV 66; N, HPV 68.
Figure 9 represents an electrode array for HPV detection. "br" is biotine marker; "ic", internal positive control "ec", external positive control; A, HPV 39; B, HPV 45; C, HPV 16; D, HPV 18; E, HPV 31; F, HPV33; G, HPV 35; H, HPV 51; I, HPV 52; J, HPV 56; K, HPV 58; L, HPV 59; M, HPV 66; N, HPV 68. A... N, immobilized capture probe (cpA...N); c, counter electrode. The superscripts indicate the respective quarter of the array.

### Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition. The definitions given herein are included for the purpose of understanding and expected to be applied throughout description, claims and drawings.

The method of the invention is for electrochemical detection of a target DNA. In the present invention "target DNA" refers to any nucleic acid molecule susceptible of being detected, DNA or RNA. Target DNA preferably is DNA. When target DNA is RNA, the amplification step is preceded by a retrotranscriptase event; e.g. RNA is amplified by rtPCR. Any method for RNA amplification known from the State of the Art by the skilled person, may be performed. The target DNA can be of any length. It can be a gene, a fragment of a gene, a non-codifying sequence, a polymorphism, a cDNA, a microRNA, a mutation, a point mutation, a deletion, an insertion. The target DNA may be purified or present in a tissue, for example a cervical smear, therefore, the target DNA may be comprised in a sample. The sample can be obtained from any subject. In a preferred embodiment the target DNA is a nucleic acid from a virus, a bacteria or a mammal, such as a mouse, rat, guinea pig, rabbit, dog, cat, bovine, horse, goat, sheep, primate or human, preferably a human. The subject may be of any age, gender or race.

In a preferred embodiment of the first aspect of the invention the target DNA from a microorganism is selected from the group consisting of: Human Papillomavirus, including High Risk (16 18 31 33 35 39 45 51 52 56 58 59 66 68), Low Risk (6 11 40 42 43 44 54 61 62 70 71 72 81 83 84 85 89) and undetermined risk genotypes: 34, 64, 67, 69 74, 86, 87, 97, 101, 102, 103, 106, 150, 151; viruses causative of respiratory infections, such as: Adenovirus; Bocavirus; Coronavirus 229E; Enterovirus; Influenza virus A (H7N9, H3N2, H1 N1 and H1N1/2009), Influenza virus B, Influenza virus C; Metapneumovirus (subtypes A and B); Parainfluenza virus 1, 2, 3 and 4 (subtypes A and B); Rhinovirus; Respiratory syncytial virus type A (RSV-A) and Respiratory syncytial virus type B (RSA-B), Coronavirus OC43, Coronavirus NL63 and Influenza A H7N9; human Herpesviruses, including Herpes Simplex Virus 1, Herpes Simplex Virus 2, Varicella Zoster Virus 3, Epstein-Barr Virus, Citomegalovirus, Human Herpes Virus 6, Human Herpes Virus 7, Human Herpes Virus 8; Enteroviruses, including Coxsackievirus, Poliovirus and Echovirus; Gram positive and Gram negative bacteria and fungi causing septicemia, such as *Staphylococcus aureus, Staphylococcus epidermidis, Staphylococcus hominis, Staphylococcus haemolyticus, Streptococcus pyogenes*/*dysgalactiae, Streptococcus pneumoniae, Streptococcus mitis, Streptococcus agalactiae, Streptococcus sanguinis*/*parasanguinis, Streptococcus milleri (including S. constellatus and S. anginosus), Streptococcus spp, Enterococcus faecium, Enterococcus faecalis,* Gram positive cocci (CGPs), *Listeria monocytogenes, Clostridium perfringens*, *Escherichia coli, Klebsiella pneumoniae, Klebsiella oxytoca, Klebsiella* spp., *Salmonella enterica, Enterobacter cloacae, Enterobacter aerogenes, Enterobacter* spp. (*E. cloacae, E. aerogenes*), *Citrobacter freundii, Citrobacter* spp. (C. *freundii, C. koserii*), *Serratia marcescens*/*plimutica, Proteus vulgaris*/*penneri, Proteus mirabilis, Proteus* spp. (P. *vulgaris, P. mirabilis, P. penneri*), *Haemophilus influenza, Haemophilus* spp. (H. *influenzae, H. parainfluenzae), Acinetobacter baumanii, Bacteroides fragilis, Bacteroides* spp. (*B. fragilis, B. faecis*), *Pseudomonas* spp (*P. aeruginosa, P. putida, P. stuartii*), *Stenotrophomonas maltophilia, Candida albicans, Candida glabrata, Candida krusei, Candida* spp., *fungal taxa*; detection of Bacteria that produce endotoxins and cause diarrhea, such as *Salmonella* spp. *,Shigella* spp. (S. *dysenteriae, S. Sonnei, S. boydii and* S. *flexneri*), *Yersinia enterocolitica, Yersinia* spp. (*Y. pestis, Y. pseudotuberculosis, Y. enterocolítica*), *Campylobacter* spp. (C. *lari, C. laridis, C. upsaliensis, C. jejuni, C. coli*), *Campylobacter jejuni, Campylobacter coli, Escherichia coli enteropathogenic* EPEC: (*E. coli* enterohemoragic, *E*. *coli* enteroinvasive, *E*. *coli* enterotoxigenic and E. *coli* enteropathogenic), *Clostridium difficile B, Aeromonas* spp, producers of aerolisin; detection and typing of bacteria, fungi and parasites causing sexually transmitted infections (STIs) of the urogenital tract, such as *Chlamydia trachomatis, Neisseria gonorrhoeae, Mycoplasma genitalium, Trichomonas vaginalis, Ureaplasma urealyticum*/*parvum, Mycoplasma hominis, Candida* (*C.albicans, C.glabrata, C.parapsilosis, C.krusei, C.tropicalis, C.guilliermondii, C.dubliniensis*), *Treponema pallidum, Haemophilus ducreyi,* Herpes Virus types I and II; detection of multiple bacteria causing respiratory tract infections, such as *Staphylococcus aureus, Streptococcus pneumoniae, Haemophilus influenzae, Haemophilus* spp, *Moraxella catarrhalis, Mycoplasma pneumoniae, Bordetella pertussis, Bordetella parapertusis, Bordetella bronchiseptica, Bordetella holmesii, Bordetella* spp. In a particular embodiment, the method of the invention is for detection and/or typing of *Human Papillomavirus.*

In another preferred embodiment of the first aspect of the invention the target DNA is a gene or fragment of the gene that can have mutations associated with response to therapy in cancer patients. Some examples include (i) point mutations in genes of the Epidermal Growth Factor Receptor (EGFR) pathway associated to colorectal cancer, such as point mutations in the KRAS gene (e.g.: G12A, G12C, G12D, G12R, G12S, G12V, G13D, Q61H, Q61L, Q61H (183 A>C), K117N (351 A>C), K117N (351 A>T), A146V, A146T), BRAF gene (e.g.: V600E,V600K), PI3K gene (E542K, E545D, E545K, H1047R), NRAS gene (G12D, Q61 H (183 A>T), Q61R, Q61 K, Q61L); (ii) point mutations, deletions and insertions in the EGFR gene (point mutations G719A (c.2156 G>C), G719C (c.2155 G>T), G719S (c.2155 G>A), S768I (c.2303 G>T), T790M (c.2369 C>T), L858R (c.2573 T>G), L861Q (c.2582 T>A); deletions 6223* E746_A750del (c.2235_2249 del 15), 12370* L747_P753>S (c. 2240_2257del18), 12369* L747_T751del (c.2240_2254del15), 6255* L747_S752del (c. 2239_2256del18), 12384* E746_S752>V (c.2237_2255>T), 12382* E747_A750>P (c.2239_2248TTAAGAGAAG>C), 6225*, 12678*, 6218*, 12728*, 6220*, 12419*, 6210*, 13556*, 12386*, 12385*, 18427*, 12403*, 12383*, 6254*, 13551*, 12367*, 12422*, 12387*, 26038*, 13552*, 12416*, 23571*; insertions H773_V774insH (c.2319_2320insCAC), D770_N771insG (c.2310_2311insGGT), V769_D770insASV (c.2307 _2308ins9GCCAGCGTG), V769_D770insASV (c.2309_2310AC>CCAGCGTGGAT), D770_N771 insSVD (c.2311_2312ins9GCGTGGACA)), which have been found associated with response to therapy in non-small-cell lung cancer patients; (iii) point mutations V600E and V600K in the BRAF gene; I111S, P124S and E203K in the MEK1 gene; and Q79K in the AKT1 gene, which have been found associated with response to therapy in patients with melanoma.

The method of the invention may be used in the detection of any of the above targets and in the diagnosis of a disease or condition associated to any of the above defined target DNAs. The invention thus provides a method for detecting any of the above defined targets and also a method for the diagnosis of a disease or condition which is associated with any of the above defined targets which comprises performing the method of the invention. In some embodiments the disease is cancer. In a preferred embodiment the cancer is cervical cancer. In other embodiments the target is a mutation in a gene associated with response to therapy in cancer patients. The devices described herein are thus also useful for the prognosis and response to therapy in cancer patients.

Step (i) of the method of the invention comprises amplifying the target DNA. Thus, a sample is provided which contains the target DNA. It is noted that the sample may comprise DNA or consist of DNA. Prior purification of the DNA may be recommendable in some cases. In a preferred embodiment of the first aspect of the invention in step (i) the primer is chemically linked to a detectable label through its 5' end. As used herein, the term "label" refers to a directly or indirectly detectable compound or composition that is chemically linked to the primer so as to generate a "labelled" primer. The label may be detectable by itself, or alternatively, the label may comprise an active site that may catalyze a chemical alteration of a substrate compound or composition which is detectable, or alternatively, the label may be recognized and bound by a label-binding molecule that comprises an active site that may catalyze a chemical alteration of a substrate compound or composition which is detectable. In some embodiments the catalytic active site is an enzyme moiety. In the sense of the present invention, the chemical alteration is the oxidation or reduction of the substrate and the enzyme moiety is an oxidoreductase enzyme. In this case, the oxidoreductase enzyme is also called "transfer mediator". The transfer mediator thus catalyses the oxidation or reduction of a substrate, which subsequently generates a detectable voltage or current signal at the electrode. Non-limiting transfer mediators or (enzyme moieties) that are compatible with the method of the invention are a peroxidase, in particular, horseradish peroxidase (HRP), an acid phosphatase, alkaline phosphatase, glucose oxidase, and other enzymes or analogues or combinations thereof.

The labels can be suitable for small scale detection but are preferably suitable for high-throughput screening. The linking of the label to the primer can be done directly or through a linker molecule. The term "linker" or "linker molecule" refers to any chemical entity that connects the label with the primer. The "linker" encompasses any modifying group added to the nucleotide backbone (in this case, the primer sequence) in order to provide a reactive group for the attachment of a label or a compound that comprises reactive groups that allow for attachment of both the nucleotide sequence and the label. In the latter case the "linker" may also be termed as a "spacer". The label is preferably covalently linked to the primer. This definition of "linker" also applies to the connection or attachment of the complementary oligonucleotide capture probe to the electrode, since said attachment may be done directly between the capture probe and the electrode or through a linker. Methods for labelling DNA as well as labels appropriate for detection of DNA by electrochemical means are known to the skilled person.

The amplification reaction in the step (i) of the first aspect of the invention is performed under the conditions necessary for the target DNA and the specific amplification primers. The skilled person is able to design appropriate primers to amplify a given target sequence. However, in order to obtain an improved sensitivity, the skilled artisan must take care that the distance between the 5'-end of the amplified DNA molecule and the first nucleotide of the hybridization region within the same amplified DNA molecule is comprised from 0 to 300 nucleotides. Designing the amplification conditions given a particular target DNA sequence is within reach of the skilled person or may be found in the prior art. Methods for amplification of nucleic acids are well known in the state of the art. Polymerase Chain Reaction (PCR) and its variants, such as Reverse Transcription PCR (RT-PCR), are the most common amplification techniques. In a preferred embodiment of the present invention the amplification is performed by PCR.

The hybridization in step (iii) of the first aspect of the invention is performed under the specific conditions for each ssDNA and its complementary oligonucleotide capture probe.

The term "hybridization" herein refers to the process in which two single-stranded polynucleotides (the denaturated ssDNA and the complementary oligonucleotide capture probe) bind non-covalently to form a stable double-stranded polynucleotide ( triple-stranded hybridization is also theoretically possible). The resulting (usually) double-stranded polynucleotide is a "hybrid." Methods for conducting polynucleotide hybridization assays have been well developed in the art. Hybridization assay procedures and conditions will vary depending on the application and are selected in accordance with the general binding methods known in the art. It is appreciated that the ability of two single stranded polynucleotides to hybridize will depend upon factors such as their degree of complementarity as well as the stringency of the hybridization reaction conditions.

As used herein, "stringency" refers to the conditions of a hybridization reaction that influence the degree to which polynucleotides hybridize. Stringent conditions can be selected that allow polynucleotide duplexes to be distinguished based on their degree of mismatch. High stringency is correlated with a lower probability for the formation of a duplex containing mismatched bases. Thus, the higher the stringency, the greater the probability that two single-stranded polynucleotides, capable of forming a mismatched duplex, will remain single-stranded. Conversely, at lower stringency, the probability of formation of a mismatched duplex is increased. Means for adjusting the stringency of a hybridization reaction are well-known to those of skill in the art.

The "hybridization region" in the present invention is referred to the oligonucleotides within the amplified, denatured ssDNA that form the hybrid between said denatured ssDNA and the complementary oligonucleotide capture probe.

The probe in the present invention is an oligonucleotide complementary to a region of the denatured ssDNA. A complementary oligonucleotide capture probe in the sense of the present invention is an oligonucleotide that is substantially complementary to the target DNA, in particular complementary to a region of the denatured ssDNA. By "substantially" complementary it is understood that the capture probe is at least 90% complementary to the hybridization region within the target sequence, preferably at least 95% complementary, for example 96%, 97%, 98%, 99% or 100% complementary to the hybridization region. The skilled person is able to design appropriate capture probes that hybridize to a given target sequence.

The complementary oligonucleotide capture probe can be designed to allow for detection of any DNA target sequence. For example, the capture probe may allow detection of point mutations and single nucleotide polymorphism, or of DNA sequences of variable size, for example from 40 to 1500 nucleotides, particularly from 50 to 500 nucleotides.

In a particular embodiment, the oligonucleotide capture probe is for detection and/or typing of human HPV. The capture probe is thus complementary to a sequence comprised in human HPV. Capture probes for detection of different human HPV types are known in the art and have been disclosed, for example, in WO2007017699, which is herein incorporated by reference. Primers for amplifying target sequences comprised in the HPV types are likely disclosed in WO2007017699.

The term "electrode" is defined as an electrically conductive substance that remains stationary during an electrochemical analysis. Examples of electrode materials include solid metals, metal pastes, conductive carbon, conductive carbon pastes, and conductive polymers. The surface of the electrode may be functionalized to facilitate attachment of the oligonucleotide capture probe. The electrode may alternatively be recovered by a polymer that also facilitates attachment of the oligonucleotide capture probe or minimizes unspecific reactions or background signals. For the method of the invention the electrodes preferably comprise Au, Pt, Ag, Ag/Cl or C. The electrode to which the at least one probe is attached is called working electrode. The method of the invention may require the presence further electrodes, namely, at least one reference electrode and at least one counter electrode. In particular embodiments the working electrodes comprise gold or carbon, preferably Au, the counter electrode comprises gold or carbon, preferably carbon, and the reference electrode comprises Ag/AgCl. In a particularly preferred embodiment, the working electrodes comprise gold, the counter electrode comprises gold, and the reference electrode comprises Ag/AgCl. The electrodes may comprise layer of different composition. For example, the electrodes may comprise a Titanium bottom layer facing the substrate and a gold top layer facing the measurement cell and to which the probe is attached. Ti is a good bonding agent between substrate and Au, while Au has been found appropriate for probe attachment. The electrode may be comprised in an array or microarray. In the present invention the terms "array", "microarray", "DNA array," "array chip," "DNA array chip," "bio-chip" or "chip" and "microarray platform" are used interchangeably. They refer to a collection of a large number of probes arranged on a shared substrate which could be a portion of a plastic surface, silicon wafer, a nylon strip or a glass slide. The microarray preferably comprises several capture probes attached to several electrodes. Each electrode may comprise at least one capture probe for detection of one target DNA (i.e. which hybridizes with one target DNA). Alternatively, one electrode may comprise more than one, for example, two, three or four, different capture probes to detect the same target DNA. The shared substrate in the case of the present invention is preferably a cartridge.

The capture probe is attached to the substrate of the array that comprises the electrode through its 3'. The attachment of the probe through its 3' end to the array platform, preferably to the electrode, can be performed through a linker (the term "linker" has been defined above). Methods for attaching or binding oligonucleotide capture probes to solid supports and in particular to electrodes for electrochemical detection are known in the art (Sensors 2014, vol. 14, p. 22208-22229). For example, thiol-metal interactions are frequently used for covalent binding of biomolecules on gold surfaces. The thiol groups demonstrate a strong affinity towards the noble metal surfaces allowing the formation of covalent bonds between the sulphur and gold atoms. On the basis of this principle (chemisorption), DNA arrays have been developed using thiol-modified DNA probes. In a particular embodiment the capture probe is thiol-functionalized at its 3'end and is attached to the electrode (in particular a gold electrode) by chemisorption.

The specific complementary oligonucleotide probe can be immobilized or attached by means of incubation, by direct synthesis on the electrode surface or by ink-jet deposition.

Step (iv) of the method of the first aspect of the invention comprises determining the presence of the label associated with the hybridization product. In a particular embodiment, this determination comprises:
a) Adding to the hybridization product a label-binding molecule to form a complex, wherein said label-binding molecule is chemically linked to an enzymatic moiety,
b) Adding to the complex resulting from step (a) a substrate which undergoes oxidation or reduction in the presence of the enzymatic moiety; and
c) determining the oxidation or reduction of the substrate.

The term "label" has been defined above. The term "label-binding molecule" refers to a molecule that has a high affinity and binds to the label through a chemical link, thus forming a complex. In a particular embodiment of the method of the invention, the label is an hapten or an antigen. Haptens are small molecules that can in certain instances be induced to elicit an immune response if they are first coupled to a large carrier (such as a protein) to form an immunogen. Haptens, as antigens, are employed in bioassays for their strong binding properties. Haptens that have been developed for analytical procedures include biotin, digoxigenin, fluorescein, damatane and carbazole. Haptens in combination with an hapten-binder are useful for detecting particular molecular targets, for example nucleic acid probes. The label-binding molecule is a molecule, such as a protein (including an anitibody), that has a strong affinity and binds the label to form a complex. In particular embodiments of the invention the label is preferably biotin or digoxigenin and the label-binding molecule is an hapten-binder, preferably selected from avidin, streptavidin, neutravidin, or any of their analogs, and an antibody (such as anti-digoxigenin antibody or anti-biotin antibody). The antibody can be monoclonal or polyclonal. In a preferred embodiment the label is biotin and the label-binding molecule is streptavidin.

The enzymatic moiety (or transfer mediator) is preferably an oxido-reductase enzyme that performs a redox reaction. The term "oxidation" means losing electron to oxidize. The term "reduction" means gaining electrons to reduce. The term "redox reaction" refers to any chemical reaction which involves oxidation and reduction. In a particular embodiment the enzymatic moiety is horse-radish peroxidase (HRP) or an acid phosphatase, preferably is HRP, more preferably is polyHRP. In a preferred embodiment the label is biotin and the label-binding molecule is streptavidin linked to HRP, more preferably to polyHRP.

In the sense of the present invention the term substrate refers to a compound or group of compounds that is oxidized or reduced in the presence of the enzyme moiety. In a particular embodiment the substrate is an electron donor substrate. Appropriate electron donor substrates are selected from 3,3',5,5'-tetramethylbenzidine (TMB), peroxide, cathechol, OPD (orthophenylene diamine), aminophenols or aromatic polyamines. In a particular embodiment the substrate is TMB. In another particular embodiment the substrate is TMB and the enzymatic moiety is HPR, preferably polyHRP.

In a preferred embodiment of the invention, the 5'-label is biotin, the label-binding molecule is selected from streptavidin, avidin and neutravidin (preferably streptavidin), the enzymatic moiety is HRP (preferably polyHRP), and the substrate is TMB.

In some embodiments, the step of determining the oxidation or reduction of the substrate comprises sensing the presence or absence of a voltage or current signal generated by the oxidation or reduction of the substrate. The step of sensing often comprises using a reference voltage or current signal to determine the voltage or current signal resulting from the oxidation/reduction of the substrate, wherein the presence of a voltage or current signal is indicative of the presence of the target DNA. A constant potential (voltage) is applied between counter electrode and working electrode, the current between both of them is the measured value. The reference electrode is necessary to control the applied potential due to its defined potential in the galvanic series. The signal is usually detected by means of a potentiostat or polypotentiostat.

In a particular embodiment of the first aspect of the invention the distance between the 5' end and the first nucleotide of the hybridization region within the ssDNA molecule to be determined is comprised from 0 to 300, more particularly from 10 to 200, more particularly from 25 to 150, more particularly from 30 to 120, more particularly from 50 to 90 (50, 51, 52, 53, 54, 55, 56, 57, 59, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89 or 90).

A second aspect of the invention refers to use of means for electrochemical DNA detection as defined above, wherein the means comprise: i) A pair of primers to amplify the DNA target, wherein at least one of the primers is chemically linked to a label through its 5' end; ii) An oligonucleotide capture probe complementary to the DNA target sequence, wherein the oligonucleotide capture probe is attached through its 3' end to an electrode; and iii) means to detect the label; Wherein when in use the distance between the 5'-end and the first nucleotide of the hybridization region within the ssDNA molecule that hybridises to the probe is comprised from 0 to 300 nucleotides

The above means, and in particular, the primers, allow to generate an amplification product wherein the distance between the 5'-end and the first nucleotide of the hybridization region within the ssDNA molecule to be determined is comprised of from 0 to 300 nucleotides, more particularly from 10 to 200, more particularly from 25 to 150, more particularly from 30 to 120, more particularly from 50 to 90, for example 50, 51, 52, 53, 54, 55, 56, 57, 59, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89 or 90.

The means may comprise more than one oligonucleotide capture probe complementary to the DNA target sequence and more than one electrode. In particular embodiments the means comprises two or more different capture probes each attached to a different (working) electrode. In another embodiment more than one different capture probe complementary to the same target sequence is attached to the same electrode. The number of electrodes is variable and may depend on the number of target DNAs to be determined. In some embodiment the means comprises from 1 to 150, in particular 10 to 100 electrodes, preferably from 30 to 80 electrodes, for example 64 electrodes.

In particular embodiments the means (iii) to detect the label comprise:
a) A label-binding molecule, wherein said label-binding molecule is chemically linked to an enzymatic moiety; and
b) A substrate which undergoes oxidation or reduction in the presence of the enzymatic moiety.

The primers, capture probes, electrodes, and other means (labels, enzyme moieties, substrates) are as described above in more detail. Other means such as buffers, and wash solutions may also be used to perform the method of the invention. The means are preferably comprised in a cartridge. Further embodiments related to the design of a cartridge of the invention are described below.

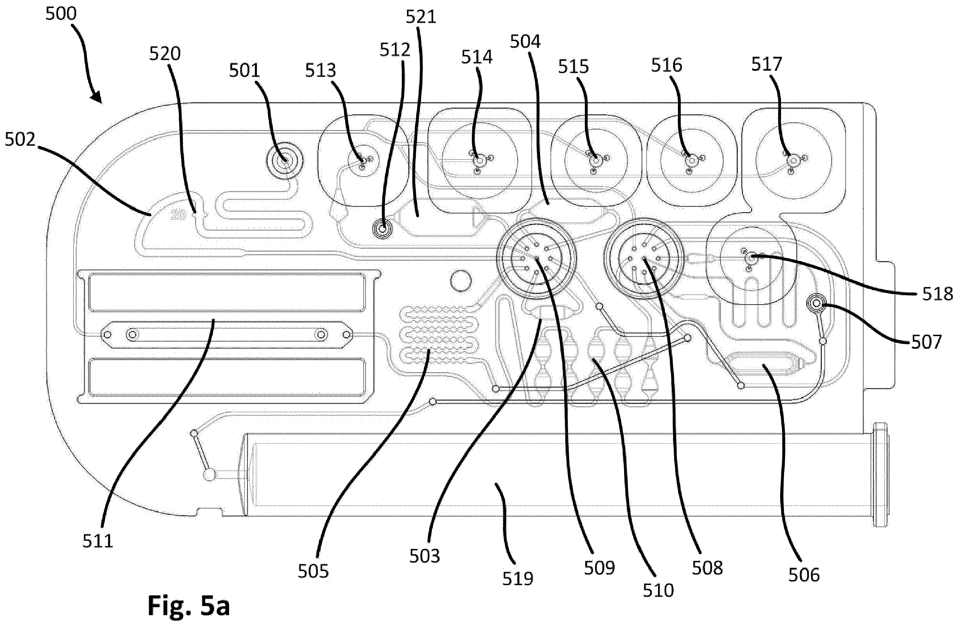

Figure 5a is a schematic representation of a cartridge device 500, according to examples, suitable for performing electrochemical DNA detection methods similar to the ones described in other parts of the disclosure.

The cartridge device 500 may comprise a sample inlet 501 for inputting a sample to be processed, and a micro-fluidic circuit. The sample may comprise DNA or consist of DNA. The sample inlet 501 may comprise, for example, a short Luer interface with cone at bottom for pipet tip or blood capillary sealing structure. This way, the inputted sample may be suitably provided to the micro-fluidic circuit.

The micro-fluidic circuit may comprise a sample metering (microfluidic) loop 502 in fluid communication through micro-pipe(s) with the sample inlet 501, and a sample metering (microfluidic) chamber 503 in fluid communication through micro-pipe(s) with the sample metering loop 502.

The sample metering loop 502 may be configured to contain, for example, a maximum fluid volume of approximately 40 µl with a loading mark 520 at approximately 20 µl. The sample metering chamber 503 may be configured to contain, for example, a maximum fluid volume of approximately 12.5 µl.

The fluid communication through micro-pipe(s) between the sample metering loop 502 and the sample metering chamber 503 may be regulated through turning valve 509, which may be operable in such a way that the sample metering chamber 503 may receive the sample from the sample metering loop 502 when required.

The cartridge 500 may further comprise a container (e.g. blister) 513 containing amplification buffer (preferably PCR buffer), a container (e.g. blister) 514 containing first wash buffer, a container (e.g. blister) 515 containing a trigger, a container (e.g. blister) 516 containing a conjugate, a container (e.g. blister) 517 containing second wash buffer, and a container (e.g. blister) 518 containing hybridization buffer. Any of the blisters may be configured to provide its content to the microfluidic circuit when properly pressed.

The micro-fluidic circuit may further comprise a PCR buffer metering (microfluidic) chamber 504 in fluid communication through micro-pipe(s) with the blister 513 containing amplification (or PCR) buffer. This fluid communication may be regulated through turning valve 509, which may be operable in such a way that the PCR buffer metering chamber 504 may receive the content of the blister 513 when required. The PCR buffer metering chamber 504 may be configured to contain, for example, a fluid volume of approximately 37.5 µl.

The micro-fluidic circuit (of the cartridge 500) may still further comprise a sample and PCR buffer mixing (microfluidic) channel 505 in fluid communication through micro-pipe(s) with the sample metering chamber 503 and the PCR buffer metering chamber 504. This fluid communication may be regulated through turning valve 509, which may be operable in such a way that the sample and PCR (or amplification) buffer mixing channel 505 may receive, and therefore mix, the content of the sample metering chamber 503 and the content of the PCR (or amplification) buffer metering chamber 504 when required.

The micro-fluidic circuit further comprises an amplification (microfluidic) chamber 506, preferably a PCR (microfluidic) chamber, in fluid communication through micro-pipe(s) with the sample and PCR buffer mixing channel 505. This fluid communication may be regulated through turning valve 508, which may be operable in such a way that the PCR chamber 506 may receive the mixture of the sample and the PCR (or amplification) buffer from the sample and PCR buffer mixing channel 505 when required. The PCR (or amplification) chamber 506 may be configured to contain, for example, a fluid volume of approximately 30 µl.

The PCR (or amplification) buffer from blister 513 may include amplification primers, wherein at least one of said primers may be chemically linked to a label (e.g. biotin) through its 5' end. DNA in the sample may thus be amplified with such primers and denatured in the PCR (or amplification) chamber 506, to obtain corresponding 5'-labelled ssDNA molecule(s). In alternative examples, the microfluidic circuit may further comprise a denaturation chamber (not shown) so that amplification and denaturation may occur in different chambers. Using a single chamber for both amplification and denaturation may permit saving energy and a more compact design of the cartridge device.

The micro-fluidic circuit may additionally comprise a PCR and hybridization buffer mixing (microfluidic) channel 510 in fluid communication through micro-pipe(s) with the PCR (or amplification) chamber 506 and the blister 518 containing hybridization buffer. This fluid communication may be regulated through turning valve 508, which may be operable in such a way that the PCR (or amplification) and hybridization buffer mixing channel 510 may receive, and therefore mix, the content of the PCR (or amplification) chamber 506 and the blister 518 when required.

The PCR and hybridization buffer mixing channel 510 may be configured to contain, for example, a fluid volume of approximately 70 µl.

The cartridge 500 may yet further comprise a syringe with plunger (or fluid flow inducer) 519 in fluid communication through micro-pipe(s) with the microfluidic circuit of the cartridge. The syringe 519 may be operable in such a way that a suction effect may occur for causing suitable fluid flow within the microfluidic circuit as required. A usable volume in the syringe (or fluid flow inducer) 519 may be, for example, approximately 6.5 ml.

The cartridge 500 may additionally comprise ventilation outlets 512 with waste (microfluidic) chamber 521 in fluid communication through micro-pipe(s) with the microfluidic circuit of the cartridge. This fluid communication may be regulated through turning valve 509, which may be operable in such a way that excess fluid from e.g. the sample metering chamber 503, the PCR buffer metering chamber 504, the sample and PCR buffer mixing channel 505, etc. may be received (and accumulated) by the waste chamber 521. This transfer of excess fluid to the waste chamber 521 may also comprise excess air in the microfluidic circuit which may thus be expelled through ventilation outlets 512.

The cartridge 500 may also comprise a stop valve 507 which may comprise e.g. a PTFE membrane that may act as a barrier when the membrane is impregnated by fluid to a certain extent. The stop valve 507 may be in fluid communication through micro-pipe(s) with the PCR chamber 506 through turning valve 508, which may be operable in such a way that loading of the PCR (or amplification) chamber 506 may be stopped by the barrier created in the stop valve 507 (when e.g. the PTFE membrane is sufficiently impregnated).

The cartridge 500 may still further comprise a sensor (microfluidic) chamber 511 in fluid communication through micro-pipe(s) with the PCR and hybridization buffer mixing channel 510, so that the sensor chamber 511 may receive the mixture of PCR (or amplification) and hybridization buffer when required. The sensor chamber 511 may be configured to contain, for example, a fluid volume of approximately 70 µl, and may include an electrode (micro) array.

The electrode (micro) array may comprise working, counter and reference electrodes with a specific complementary oligonucleotide probe immobilized thereto at its 3' end. Hybridization of at least one biotin-labelled ssDNA molecule (from amplification in PCR or amplification chamber 506) with such an oligonucleotide probe may thus occur in the sensor chamber 511. In said hybridization process, the distance between the 5' biotin and the hybridization region to the complementary probe within the hybridizing ssDNA molecule is of approximately 300 nucleotides or lower. In particular, the distance is comprised from 0 to 300 nucleotides, more particularly from 10 to 200, more particularly from 25 to 150, more particularly from 30 to 120, more particularly from 50 to 90 (50, 51, 52, 53, 54, 55, 56, 57, 59, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89 or 90). Preferably the distance is of approximately 85 nucleotides or lower. Further details about these considerations are provided in other parts of the disclosure.

The blister 515 containing a trigger and the blister 516 containing a conjugate may also be in fluid communication through micro-pipe(s) with the sensor chamber 511. This way, the sensor chamber 511 may receive when required the trigger and conjugate from corresponding blister 515, 516 as a result of suitable pressure exerted on the blister.

The conjugate may comprise, for example, streptavidin or analogue substance coupled to an enzymatic moiety (e.g. HRP solution). The trigger may comprise, for example, an electron donor substrate such as TMB. Detection of hybridization may occur in the sensor chamber 511 through incubation with the conjugate, followed by incubation with the trigger to cause enzymatic oxidation. Further details about these considerations are provided in other parts of the disclosure.

The aforementioned hybridization conditions (distance of 300 nucleotides or lower, or 85 nucleotides or lower) may cause the enzymatic oxidation to induce current intensities in (between) the electrodes that are significantly higher in comparison to prior art methods and devices. Therefore, methods and devices according to the present disclosure may provide much more reliable results in DNA detection. Further details about these considerations are provided in other parts of the disclosure.

The blisters 514, 517 containing wash buffer may be in fluid communication through micro-pipe(s) with the sensor chamber 511, so that the sensor chamber 511 may be washed when required upon reception of the wash buffer from blister 514, 517 as a result of suitable pressure exerted on the blister.

Washing(s) of the sensor chamber 511 may be caused between hybridization and incubation with the conjugate, and further washing(s) of the sensor chamber 511 may be caused between incubation with the conjugate and incubation with the trigger (to cause final enzymatic oxidation). Said washings may reduce disturbing background signals/currents (in the electrodes) and therefore further improve DNA detection. Further details about these considerations are provided in other parts of the disclosure.

The cartridge 500 may be fabricated in the form of an injection molded micro-structured device in thermoplastic polymers, for example.

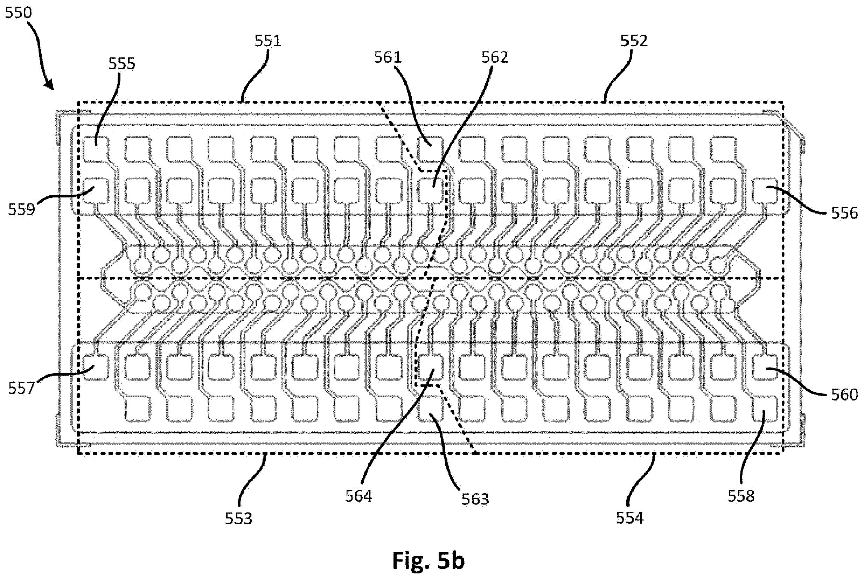

Figure 5b is a schematic representation of an electrode (micro) array 550 suitable for cartridge devices similar to the one illustrated by Figure 5a, according to examples. As explained in other parts of the description, the microarray 550 may function as a polypotentiostat.

The electrode microarray 550 may be configured to be arranged in sensor chamber 511 (see Figure 5a), and may comprise a plurality of electrodes with different functions. Some or all of said electrodes may be replicated to ensure reproducibility of the results.

In this sense, the electrode microarray 550 may be structured in first quarter of electrodes 551, second quarter of electrodes 552, third quarter of electrodes 553 and fourth quarter of electrodes 554. Other configurations with a number of parts different from four (quarters) are also possible, such as e.g. three thirds or two halves.

Specific complementary oligonucleotide probe(s) may be immobilized to electrodes of the microarray 550. This immobilization is preferably performed through a thiol modification at the 3' end of the probe. Alternative modifications of the 3' end known in the art, and/or direct synthesis of the probe on corresponding surfaces are also possible.

The electrode microarray 550 may comprise a counter electrode made of e.g. carbon and/or titanium/silver. In particular, an active part of the electrode, i.e. that part which is in contact with the immobilized probe(s), may be made of silver. As shown in the particular example of Figure 5b, first version of counter electrode 559 may exist in first quarter 551 and second version of counter electrode 560 may exist in fourth quarter 554.

The electrode microarray 550 may further comprise a reference electrode made of e.g. silver and/or silver chloride. As shown in the particular example illustrated, the reference electrode may have a first version 555 in first quarter 551, a second version 556 in second quarter 552, a third version 557 in third quarter 553, and a fourth version 558 in fourth quarter 554.

The electrode microarray 550 may still further comprise an external control electrode. In the particular example shown, the external control electrode may have a replica 562 in the first quarter and a further replica 563 in the third quarter.

The electrode microarray 550 may still further comprise an internal control electrode. In the example shown in the figure, the internal control electrode may have two versions, one version 561 in the second quarter and a further version 564 in the fourth quarter.

The electrode microarray 550 may additionally comprise a plurality of working (or measurement) electrodes made of e.g. gold and/or titanium/silver. In particular, an active part of the electrode, i.e. that part which is in contact with the immobilized probe(s), may be made of gold. The working electrodes are those electrodes different from the ones discussed above with reference to Figure 5b, and may be duplicated, triplicated or even quadruplicated (as shown in the figure) to ensure reproducibility of the results. Some working electrodes may comprise more than one attached probe(s), for example two different probes, that target the same DNA. The number of electrodes is variable and may depend on the number of target DNAs to be determined. In some embodiment the electrode microarray comprises from 1 to 150, in particular 10 to 100 electrodes, preferably from 30 to 80 electrodes, for example 64 electrodes.

Counter, reference and working electrodes may cooperate to function together as a three electrode measurement cell or potentiostat. A constant potential (voltage) may be applied between counter and working electrodes, so that electrical current(s) between them may result induced as explained in more detail in other parts of the disclosure.

These induced currents may be read and said readings may be processed to generate DNA detection results interpretable by e.g. qualified staff (e.g. doctors, nurses, etc.). Reference electrode may be used to control and adjust the constant potential applied between counter and working electrodes. Configurations including a common counter electrode, a common reference electrode and a plurality of (e.g. 64) working electrodes are known as polypotentiostat.

The (different) probe(s) may be immobilized to all the working electrodes under a diversity of relationships, such as e.g. one-to-one, many-to-one, one-to-many, depending on the detection to be performed. One-to-one relationship means that one probe is immobilized to one working electrode. Many-to-one relationship means that various different probes are immobilized to one working electrode. One-to-many relationship means that one probe is immobilized to various different working electrodes.

External and internal control electrodes are working electrodes used to check whether the means for electrochemical DNA detection (comprised in the cartridge) worked as expected and the obtained measurements (or readings) may be considered valid.

Electrode microarray may be arranged (spotted, printed) on a suitable substrate. Counter and working electrodes may be made of titanium at their bottom layer contacting the substrate, and gold at their top layer (i.e. layer opposite to the bottom layer). Titanium is a good bonding agent between the substrate and gold. Gold offers good features for immobilizing the probe(s) thereto and provides good electrical conductivity. Reference electrode may be made of silver and/or silver chloride, since these materials permit good control of the applied potential due to their defined potential in galvanic series.

Working electrodes may be spotted with a diameter of e.g. approximately 1 mm. Reference electrode may be e.g. approximately 32 mm long and approximately 1.8 mm wide.

Different types of probes may be immobilized to working electrodes. Examples of such probes may be HPV-DNA probes, control-DNA probes, biotin, etc. HPV-DNA probes may be used to detect HPV (14 different genotypes, quadruplicates each). The other cited probes may be used to perform a further control to ensure the functionality/connectivity of the array/potentiostat and check assay reagents independently from an amplified DNA.

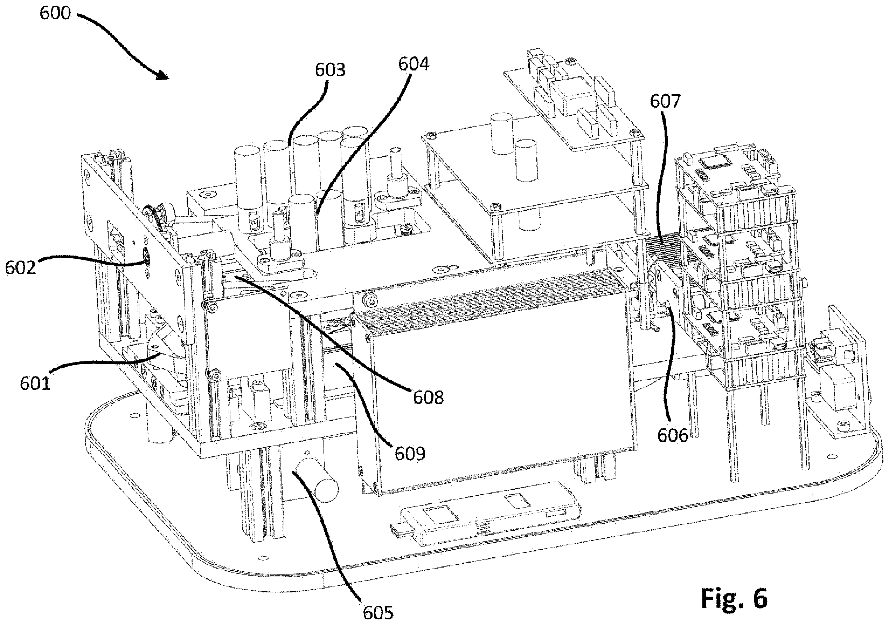

Figure 6 schematically illustrates an operator device 600, according to examples, suitable for operating cartridge devices similar to those described with reference to Figures 5a and 5b. Number references from said Figures 5a and 5b may thus be re-used in following descriptions about operator device 600.

The operator device 600 may comprise a controller (not shown) configured to control (by sending/receiving control signals to/from) different components of the operator device 600, for causing performance of DNA detection methods (as described in other parts of the disclosure) in the cartridge device 500.

The operator device 600 may further comprise a tray 601 configured to suitably receive and interface the cartridge device 500, and (optionally) a clamping mechanism 602 configured to suitably fasten the cartridge device 500 for its proper operation by the operator device 600.

The operator device 600 may still further comprise blister motors 603 which are operable (by the controller) for causing suitable pressure to any of the blisters 513 - 518 of the cartridge device 500, and turning valve motors 604 that are operable (by the controller) for suitably regulating any of the turning valves 508, 509 of the cartridge device 500.

A blister motor 603 may be operated for causing exertion of pressure to corresponding blister 513 - 518 for providing, when required, the content of the blister to the micro-fluidic circuit of the cartridge device 500. A turning valve motor 604 may be operated so as to regulate the fluid communication (between e.g. micro-channels, microfluidic chambers, etc.) within the micro-fluidic circuit of the cartridge device 500.

The operator device 600 may yet further comprise a detection unit 605 that may be operable (by the controller) for causing suitable contact between a reading contactor (comprised in the detection unit 605) and electrodes of the (micro) array 550 of the cartridge device 500. This contact between reading contactor and electrodes may cause reading of current intensities induced in the electrodes as a result of DNA detection method(s) performed in the cartridge device 500. These readings may be received and processed by the controller in order to provide interpretable results to e.g. medical and/or nursing personnel.

The operator device 600 may also comprise a driver 606 of the fluid flow inducer 519 operable (by the controller) to drive the syringe (or fluid flow inducer) 519 of the cartridge device 500, in order to cause, when required, a suction and consequent fluid motion (or flow) within the micro-fluidic circuit of the cartridge device 500. The driver 606 may comprise syringe pump motor and drive mechanism, for example.

The operator device 600 may additionally include a first heater unit 607 operable by the controller, in order to provide first heating (or incubation) cycle(s) to the PCR (or amplification) chamber 506, so that amplification and denaturation occur in the PCR chamber 506 (of the cartridge device 500).

The operator device 600 may furthermore comprise a second heater unit 608 operable by the controller for providing second heating (or incubation) cycle(s) to the sensor chamber 511 (of the cartridge device 500), so that hybridization occurs in the sensor chamber 511.

The second heater unit 608 may be also operable by the controller for providing further heating (or incubation) cycle(s) to the sensor chamber 511, so that incubation of HRP or polyHRP solution (label-binding molecule chemically linked to enzymatic moiety) contained in sensor chamber 511 is caused.

The operator device 600 may additionally comprise a lighting unit 609 for providing required illumination during operation of the cartridge device 500. The lighting unit 609 may comprise, for example, LEDs aimed at illuminating certain parts of the cartridge for visual inspection by medical/nursing staff. At least some of said LEDs may have the function of providing light indicators of determined events or situations occurred during operation of the cartridge device 500.

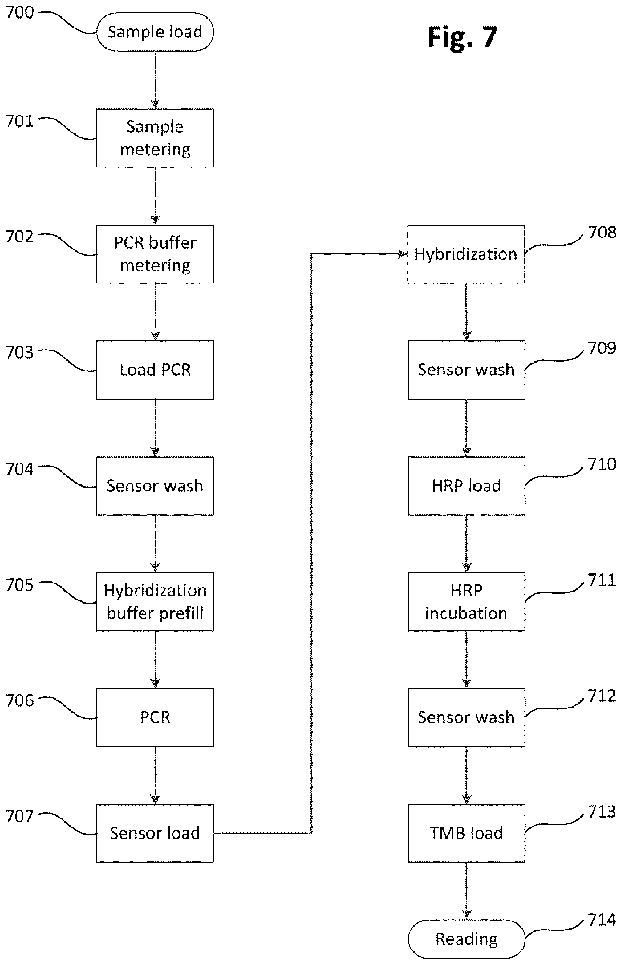

Figure 7 is a flowchart schematically illustrating a method of controlling, according to examples, an operator device 600 similar to the ones illustrated by Figure 6. Number references from Figures 5a, 5b and 6 may be re-used in following descriptions about said operating method(s).

This method may be performed by the controller of the operator device 600.

The controller (of the operator device 600) may be implemented by computing means, electronic means or a combination thereof. The computing means may be a set of instructions (that is, a computer program) and then the controller may comprise a memory and a processor, embodying said set of instructions stored in the memory and executable by the processor. The instructions may comprise functionality to execute at least a method of controlling the operator device as described in other parts of the disclosure.

In case the controller (of the operator device 600) is implemented only by electronic means, the controller may be, for example, a CPLD (Complex Programmable Logic Device), an FPGA (Field Programmable Gate Array) or an ASIC (Application-Specific Integrated Circuit).

In case the controller (of the operator device 600) is a combination of electronic and computing means, the computing means may be a set of instructions and the electronic means may be any electronic circuit capable of implementing the corresponding step or steps of the cited method of controlling the operator device 600 (for operating a cartridge device 500).

The computer program may be embodied on a storage medium (for example, a CD-ROM, a DVD, a USB drive, on a computer memory or on a read-only memory) or carried on a carrier signal (for example, on an electrical or optical carrier signal).

The computer program may be in the form of source code, object code, a code intermediate source and object code such as in partially compiled form, or in any other form suitable for use in the implementation of the method of controlling the operator device 600. The carrier may be any entity or device capable of carrying the computer program.

For example, the carrier may comprise a storage medium, such as a ROM, for example a CD ROM or a semiconductor ROM, or a magnetic recording medium, for example a hard disk. Further, the carrier may be a transmissible carrier such as an electrical or optical signal, which may be conveyed via electrical or optical cable or by radio or other means.

When the computer program is embodied in a signal that may be conveyed directly by a cable or other device or means, the carrier may be constituted by such cable or other device or means.

Alternatively, the carrier may be an integrated circuit in which the computer program is embedded, the integrated circuit being adapted for performing, or for use in the performance of, the relevant methods.

At initial block 700, loading of DNA sample through sample inlet 501 may be detected.

At block 701, control signal(s) may be sent (by the controller) to the driver 606 of the fluid flow inducer 519 for causing suction effect and consequent fluidic transfer of DNA sample from sample metering loop 502 to sample metering chamber 503.

At same block 701, control signal(s) may be further sent to turning valve motor 604 for causing the valve 509 to enable required fluid communication between sample metering loop 502 and sample metering chamber 503.

Upon filling of the sample metering chamber 503 with DNA sample, determination of whether enough quantity of DNA sample has been loaded may be performed.

At block 702, control signal(s) may be sent (by the controller) to blister motor 603 for causing pressure to the blister 513 containing PCR (or amplification) buffer, so that fluidic transfer of the PCR buffer from the blister 513 to the PCR (or amplification) buffer metering chamber 504 may occur.

At same block 702, control signal(s) may be further sent to turning valve motor 604 for causing the valve 509 to enable required fluid communication between the blister 513 and the PCR buffer metering chamber 504.

Upon filling of the PCR buffer metering chamber 504 with PCR buffer, determination of whether enough quantity of PCR (or amplification) buffer is available may be performed. Excess PCR buffer may be collected by waste chamber 521 and excess air may be expelled through ventilation outlets 512.

Control signal(s) may be also sent to the driver 606 of the fluid flow inducer 519 for causing suction effect and consequent emptying of micro-channels containing waste or excess fluid.

At block 703, control signal(s) may be sent (by the controller) to the driver 606 of the fluid flow inducer 519 for causing suction effect and consequent fluidic transfer of DNA sample (from sample metering chamber 503) and PCR buffer (from PCR buffer metering chamber 504) to the sample and PCR buffer mixing channel 505. Control signal(s) may be further sent to turning valve motor 604 for causing the valve 509 to enable required fluid communication from the sample metering chamber 503 and PCR buffer metering chamber 504 towards the sample and PCR buffer mixing channel 505.

At same block 703, subsequent control signal(s) may be sent (by the controller) to the driver 606 of the fluid flow inducer 519 for causing suction effect and corresponding fluidic transfer of mixed DNA sample and PCR buffer from sample and PCR buffer mixing channel 505 to PCR chamber 506. Control signal(s) may be also sent to turning valve motor 604 for causing the valve 508 to enable required fluid communication from the sample and PCR buffer mixing channel 505 towards the PCR chamber 506. Execution of block 703 may thus result in the PCR (or amplification) chamber 506 loaded with mixture of DNA sample and PCR (or amplification) buffer.

Further control signal(s) may be sent (by the controller) to the driver 606 of the fluid flow inducer 519 for causing suction effect and consequent emptying of micro-channels containing waste or excess fluid.

At block 704, control signal(s) may be sent (by the controller) to blister motor 603 for causing pressure to the blister 517 containing wash buffer, such that fluidic transfer of the wash buffer from the blister 517 to the sensor chamber 511 may occur. Execution of block 704 may thus result in sensor chamber 511 washed with wash buffer from the blister 517.

Further control signal(s) may be sent (by the controller) to the driver 606 of the fluid flow inducer 519 for causing suction effect and consequent emptying of the sensor chamber 511.

At block 705, control signal(s) may be sent (by the controller) to blister motor 603 for causing pressure to the blister 518 containing hybridization buffer, so that prefill with hybridization buffer from the blister 518 may occur. Control signal(s) may be also sent to turning valve motor 604 for causing the valve 508 to retain hybridization buffer from blister 518 until required.

At block 706, control signal(s) may be sent (by the controller) to first heater unit 607 for causing successive heating (and cooling) cycle(s) in the PCR chamber 506, so that PCR (or amplification) process is developed. These heating (and cooling) cycles may comprise e.g. a first cycle at 98 °C five minutes, 45 cycles at 98 °C 15 seconds, 55 °C 15 seconds and 72 °C 30 seconds, finishing with 72 °C 1 minute. An overall PCR (or amplification) time may be less than or equal to 45 minutes. The first heater unit 607 may provide required temperatures to the PCR chamber 506 in such a way that fluid expansion due to varying temperatures is substantially avoided or at least attenuated.

At block 707, control signal(s) may be sent (by the controller) to turning valve motor 604 for causing the valve 508 to enable fluid communication from the blister 518 containing hybridization buffer (retained at previous block 705) and PCR chamber 506 towards the PCR and hybridization buffer mixing channel 510. Control signal(s) may be also sent to blister motor 603 for causing pressure to the blister 518 for promoting fluidic transfer of the hybridization buffer from the blister 518 to the PCR and hybridization buffer mixing channel 510.

Once PCR fluid (from PCR chamber 506) and hybridization buffer (from blister 518) have been mixed in the PCR and hybridization buffer mixing channel 510, the resulting mixture may continue to flow towards the sensor chamber 511. Block 707 may therefore result in the filling of the sensor chamber 511 with the mixture of PCR fluid and hybridization buffer.

At block 708, control signal(s) may be sent (by the controller) to the second heater unit 608 for providing second heating cycle(s) and, therefore, causing incubation of the mixture of PCR fluid and hybridization buffer contained in the sensor chamber 511. Hybridization of the PCR fluid (from PCR or amplification chamber 506) may therefore result in the sensor chamber 511.

At block 709, control signal(s) may be sent (by the controller) to the driver 606 of the fluid flow inducer 519 for causing suction effect and consequent emptying of the sensor chamber 511.

Further control signal(s) may be sent (by the controller) to blister motor 603 for causing pressure to the blister 514 containing wash buffer, such that fluidic transfer of the wash buffer from the blister 514 to the sensor chamber 511 may occur. The sensor chamber 511 may thus result washed with the wash buffer from blister 514.

Still further control signal(s) may be sent (by the controller) to the driver 606 of the fluid flow inducer 519 for causing suction effect and consequent emptying of the sensor chamber 511.

Yet further control signal(s) may be sent (by the controller) to blister motor 603 for causing pressure to the blister 517 containing wash buffer, such that fluidic transfer of the wash buffer from the blister 517 to the sensor chamber 511 may occur. The sensor chamber 511 may thus result washed with the wash buffer from blister 517.

Additional control signal(s) may be sent (by the controller) to the driver 606 of the fluid flow inducer 519 for causing suction effect and consequent emptying of the sensor chamber 511.

At block 710, control signal(s) may be sent (by the controller) to blister motor 603 for causing pressure to the blister 516 containing conjugate (e.g. HRP solution), so that fluidic transfer of the conjugate solution from the blister 516 to the sensor chamber 511 may occur. That, is an electrochemical solution (HRP solution) is injected into the sensor chamber 511.

At block 711, control signal(s) may be sent (by the controller) to the second heater unit 608 for causing incubation of the HRP solution contained in the sensor chamber 511. Said control signal(s) may be such that incubation of the HRP solution may be performed e.g. at 37 °C for approximately 60 minutes.

At block 712, control signal(s) may be sent (by the controller) to the driver 606 of the fluid flow inducer 519 for causing suction effect and consequent emptying of the sensor chamber 511.

Further control signal(s) may be sent (by the controller) to blister motor 603 for causing pressure to the blister 514 containing wash buffer, such that fluidic transfer of the wash buffer from the blister 514 to the sensor chamber 511 may occur. The sensor chamber 511 may thus result washed with the wash buffer from blister 514. Still further control signal(s) may be sent (by the controller) to the driver 606 of the fluid flow inducer 519 for causing suction effect and consequent emptying of the sensor chamber 511.

Yet further control signal(s) may be sent (by the controller) to blister motor 603 for causing pressure to the blister 517 containing wash buffer, such that fluidic transfer of the wash buffer from the blister 517 to the sensor chamber 511 may occur. The sensor chamber 511 may thus result washed with the wash buffer from blister 517.

Additional control signal(s) may be sent (by the controller) to the driver 606 of the fluid flow inducer 519 for causing suction effect and consequent emptying of the sensor chamber 511.

At block 713, control signal(s) may be sent (by the controller) to blister motor 603 for causing pressure to the blister 515 containing trigger (e.g. TMB), so that fluidic transfer of the trigger from the blister 516 to the sensor chamber 511 may occur. That is, a detection solution (TMB) is injected into the sensor chamber 511.

At final block 714, control signal(s) may be sent (by the controller) to the detection unit 605 for causing suitable contact between reading contactor (of the detection unit 605) and the electrodes of the (micro) array 550 in the sensor chamber 511. This contact between the reading contactor and electrodes may cause reading of current intensities induced in the electrodes. These readings may be received and processed by the controller in order to provide interpretable results to e.g. medical and/or nursing personnel.

As mentioned above for the method of the invention, the devices of the invention may be used in the detection of any target DNA and, in particular for the detection of any of the target DNA that have been disclosed above. By target DNA within the invention, it is meant target nucleic acid, thereby, RNA targets also being susceptible of being detected according to it. A retrotranscriptase event preceding the amplification step would then be required. The devices of the invention also may be used in the diagnosis of a disease or condition associated to any of the above defined target DNAs. The invention thus provides methods for detecting any of the above defined targets and also methods for the diagnosis of a disease or condition which is associated with any of the above defined targets which comprises using the devices of the invention. In some embodiments the target is HPV. In some embodiments the disease is cancer. In a preferred embodiment the cancer is cervical cancer. In other embodiments the target is a mutation in a gene associated with response to therapy in cancer patients. The devices described herein are thus also useful for the prognosis and response to therapy in cancer patients.

In as far as the sample to be analyzed comprise nucleic acids, characterization of such a sample may be performed in the context of the proposed detection methods and devices, comprising nucleic acid amplification and denaturation of amplification products followed by specific probe hybridization.

As used in the specification and claims, the singular forms "a", "an" and "the" include plural references unless the context clearly dictates otherwise. For example, the term "an array" may include a plurality of arrays unless the context clearly dictates otherwise.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

### Example 1: PCR and hybridization assay

### Process steps of the assay:

1. Sample uptake (sample comprising DNA or purified DNA)
2. Sample transfer on chip
3. Close cap
4. Meter sample
5. Meter PCR buffer
6. Transfer sample and buffer through mixing channel into PCR chamber with lyophilized PCR reagents
7. PCR
8. Mix sample from PCR with metered volume of hybridization buffer
9. Prewash sensor
10. Flush sensor area with sample
11. Hybridization
12. Sensor wash steps (3x)
13. Flush sensor area with electrochemical solution
14. Incubation
15. Sensor wash steps (3x)
16. Add detection solution (substrate)
17. Detection

### Cartridge

The overall cartridge consists of the consumable (with PCR and hybridization cavities), the biological reagents, and the electrode array.

The microfluidic cartridge will have an interface for sample uptake and will have embedded the electrochemical sensor.

Functional elements on the cartridge with PCR and hybridization assay functionality:
- 1.: Fluidic interface
- 2.: Cap to close the chip
- 3.: Blisters for reagents storage
- 4.: Valves for metering
- 5.: Mixer
- 6.: PCR unit
- 7.: Hybridization unit
- 8.: Sensor package with electrodes
- 9.: Fluidic interfaces to the instrument
- 10.: Pneumatic interfaces to the instrument
- 11.: Thermal interface to the instrument (ensure defined temperature)
- 12.: Electrodes
- 13.: Electric interface to the instrument
- 14.: Waste reservoir

### Thermal interface - PCR

In order to achieve a fast thermocycling - in particular the cooling - this area should be as small as possible.

The requirements for the thermal interface for the PCR are summarized:
- Temperature range: room temperature to 100°C
- Temperature overshoot: max ±2°C for 5 s
- Accuracy temperature: <= ±0.5°C
- Heating rate: 2-4°C/sec (depends on final design and size of heating zone)
- Cooling rate: 2-3°C/sec (depends on final design and size of heating zone)

### Thermal interface - Hybridization area

For hybridization a constant temperature is required. There is no need to cycle the temperature. Temperature can be of 37°C.

Thermal interface - hybridization area:
- Temperature range: room temperature to 70°C
- Accuracy temperature: <= ±1°C
- Heating rate: not important since only 1 temperature need to be reached and hold
- Cooling rate: not important since only 1 temperature needs to be reached and held.

### Example 2. Procedure of functionalizing the array:

The procedure of functionalizing the array comprises the steps of:
Purchasing thiol modified probes, wherein the thiol modification is placed at the 3' end of the probe. These probes constitute the Capture probes (CP).

Deprotection of the CP through incubation with a reducing agent, which breaks disulphide groups of the thiol modifications, and yields activated versions of the CP. Next, the activated CP versions are incubated with Maleimide, and next with the array of electrodes, the later previously functionalized through incubation with a high molecular weight polyether that forms complexes with metallic ions.

Prior to incubation with the probes, the array surface is cleaned with piranha acid (mixture of H₂SO₄ and H₂O₂).

For a 64 electrode cartridge, spotting of 180nl per spot, at 60-65% rH and room temperature is required.

### Example 3: Detection of Human Papilloma Virus:

Detection and genotyping of Human Papilloma virus (HPV) 16, 18, 31, 33, 35, 39, 45, 51, 52, 56, 58, 59, 66 and 68 was performed. Those HPV types are associated with high risk of developing cervical-uterine cancer.

The detection was performed directly from samples of the uterine cervix. The samples were taken with cotton swabs and resuspended with saline buffer.

In the cartridge, specifically designed for this diagnosis, a quantity of 0.15 ml of sample resuspended with saline buffer is added.

The cartridge was introduced in the equipment previously programed with the protocol to make this type of diagnosis. The protocol took one hour fifteen minutes to be completed. The final result was an electrochemical read-out.

The array was initially functionalized as in Example 2 above.
10 µl of the sample was mixed with 30 µl of the PCR buffer and injected to the PCR chamber where the lyophilized PCR mix was stored.

### PCR:

The PCR conditions and reagents used are summarized below.

**Table 1: cycling protocol**

| **Step** | **No. of cycles** | **Temperature** | **Time** |
|---|---|---|---|
| 1 | 1 | 98°C | 300 s |
| 2 | 45 | 98°C | 5 s |
| | | 50°C | 2 s |
| | | 68°C | 25 s |
| 3 | 1 | 72°C | 60 s |

This protocol can be optimized to reach an overall PCR time of ≤ 45 minutes.

Reagents for PCR:

**Table 2: PCR reagents**

| **No.** | **Component** | **Volume** | **Conc.** |
|---|---|---|---|
| 1 | Sample | 10 µl | |
| 2 | Polymerase | | 0.1 U/µl |
| 3 | Buffer | | 1x |
| 4 | Primers | | 0.4 µM |
| 5 | Inner Control DNA | | |

These reagents/volumes can be optimized. The buffer was removed from the master mix and stored on chip (liquid). The other components were lyophilized on chip (solid).

Primers and probes from WO2007/017699A2 were used.

During PCR the right position of the sample inside the PCR chamber needs to be controlled, since changes of temperature might expand the liquid.

Once the PCR was finished, 30 µl of the amplified product was mixed with 30 µl of hybridization solution.

This mixture was denatured at 99°C for at least 2 minutes, after which it was injected to the electrochemical array and left to incubate at 37°C for 5 minutes (hybridization).

Once hybridization was finished washing steps were performed.

To amplify the signal, an electrochemical solution (polyHRP) was injected to the electrochemical array and was left to incubate at 37°C for 2 min. 30 secs.

After some washing steps of the array chamber, detection solution (TMB) was injected into the array, and hybridization was detected by electrochemistry.

Further details of the Hybridization assay and detection are provided below:
The hybridization assay was carried out at 37°C. The hybridization buffer needed to be preheated.

Description hybridization assay:
1. Once PCR was finished 30 µl of it was mixed with 30 µl of hybridization solution (SSC-Triton 1 %) that needed to be preheated at 37 °C.
2. This mixture was injected to the electrochemical array and was left to incubate at 37°C for 30 minutes (hybridization).
3. Once hybridization was finished washing solution was injected in the array chamber three times.
4. To amplify the signal, an electrochemical solution (polyHRP) was injected to the electrochemical array and was left to incubate at 37°C for 2 min 30 sec.
5. Washing solution (Tween 20 (10%) was injected in the array chamber three times.
6. Then detection solution (TMB) was injected into the array, and hybridization was detected by electrochemistry.

### Results:

The electrochemical read-out was obtained from a polypontenciostate which realized a chronoamperometry at a constant potential (-0.2V).

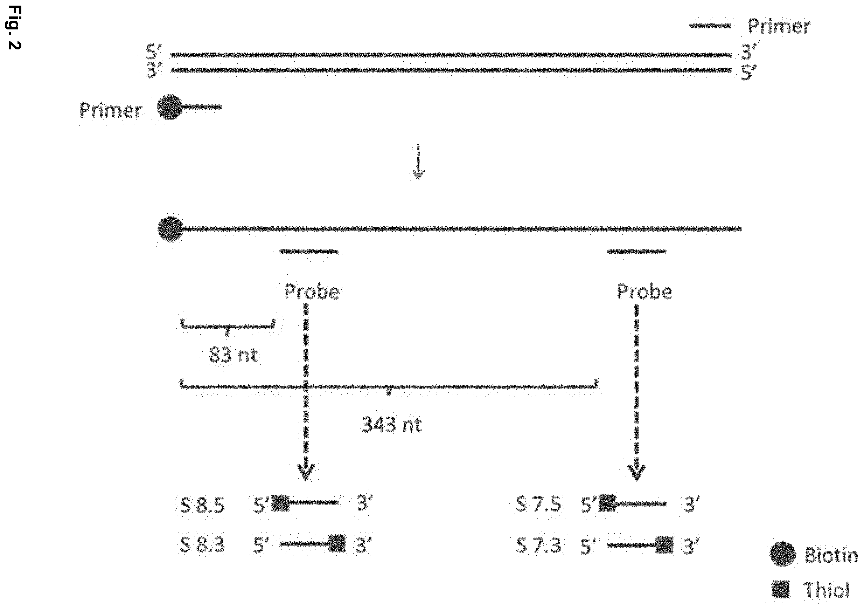

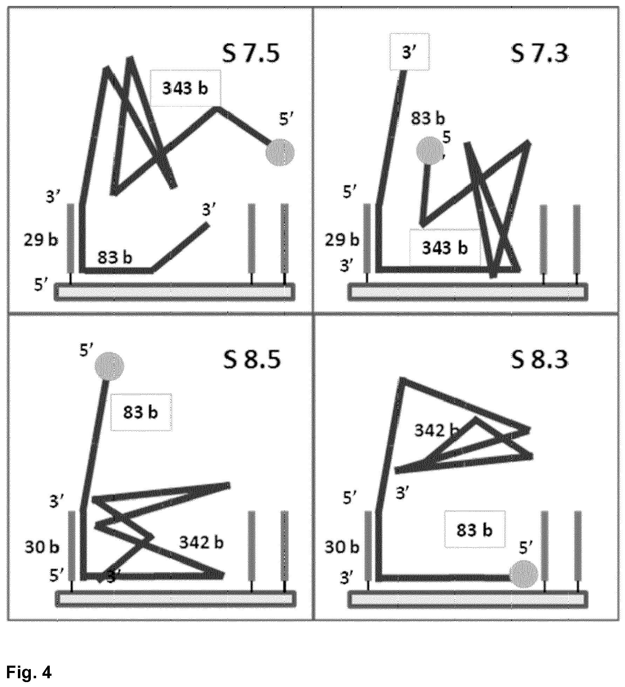

Experiments with the probe attached to the electrode through its 3' end *versus* to its 5' end were performed and with different distances between the 5' end of the primer and the hybridization region (see figures 2, 3 and 4).

In figures 2, 3 and 4, S 8.5 is a probe with thiol in its 5' end and wherein the distance between the 5' end of the primer and the hybridization region is 83 nucleotides; S 8.3, is a probe with thiol in its 3' end wherein the distance between the 5' end of the primer and the hybridization region is 83 nucleotides; S 7.5 is a probe with thiol in its 5' end wherein the distance between the 5' end of the primer and the hybridization region is 343 nucleotides; and S 7.3, probe with thiol in its 3' end and wherein the distance between the 5' end of the primer and the hybridization region is 343 nucleotides.

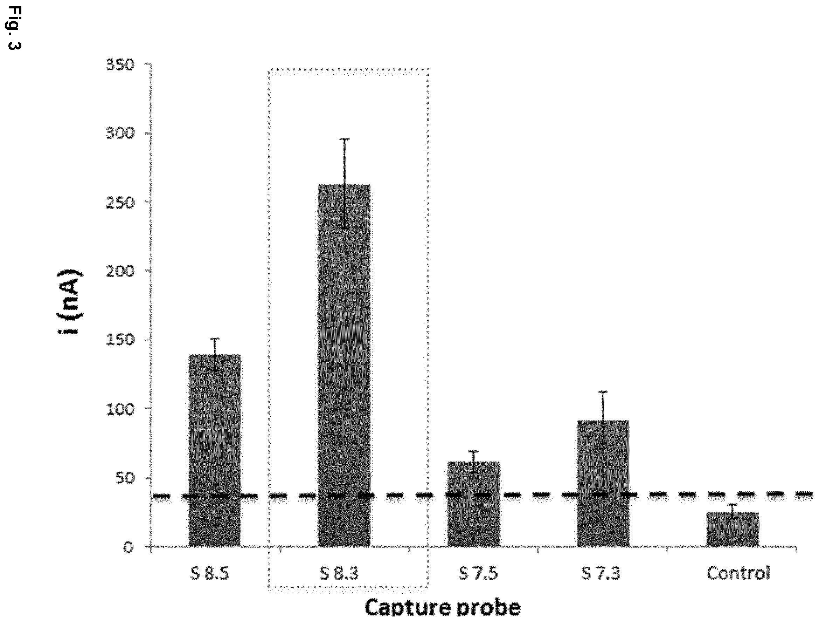

As explained in figure 3 when the probe was attached to the electrode by a thiol through its 3' end a surprising increase of electrochemical current was observed compared to that of the probe attached through its 5' end. This effect was increased when the distance between the 5' end of the primer and the hybridization region was less of than 300 nucleotides (see figure 3, results for S 8.3, compared to S 7.3).

In all the cases the primers where labelled with biotin and detected by strepavidin bound to polyHRP, the substrate used was TMB.

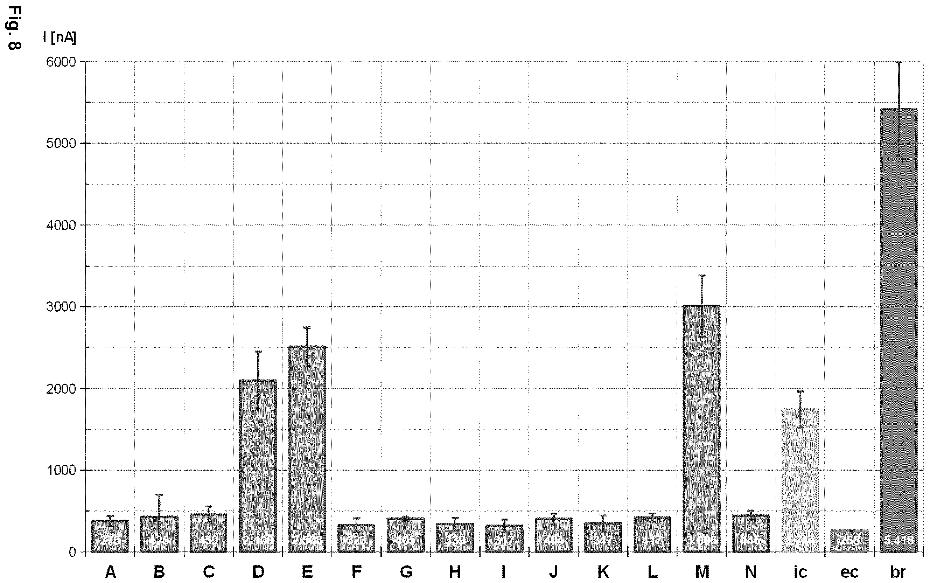

In table 3 electrochemical currents are described in each corresponding electrodes for the HPV diagnosis. Four replicates were performed. These results are also represented in Figure 8.

In table 3 "br" is biotine marker (a probe labeled with biotine impressed in the electrode array that is used to know that the polyHRP and the TMB functioned correctly); "ic", internal control of amplification (specific probe to detect the amplification of a synthetic DNA fragment, therefore to know that the PCR buffers were functioning correctly); "ec",endogenous control (specific probe to detect a human housekeeping gene present in all samples); A, HPV 39; B, HPV 45; C, HPV 16; D, HPV 18; E, HPV 31; F, HPV33; G, HPV 35; H, HPV 51; I, HPV 52; J, HPV 56; K, HPV 58; L, HPV 59; M, HPV 66; and N, HPV 68.

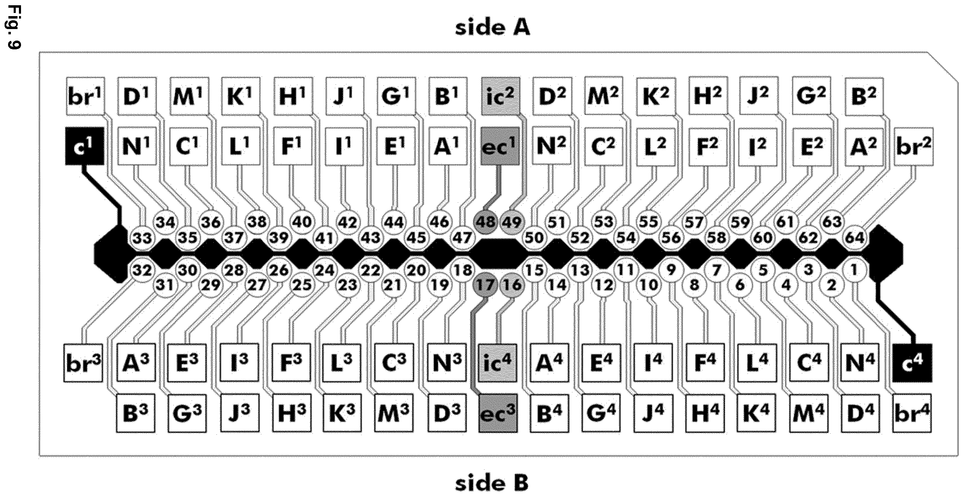

Figure 9 contains a graphical representation of the disclosed electrode array.

**Table 3:**

| | **A** | **B** | **C** | **D** | **E** | **F** | **G** | **H** | **I** | **J** | **K** | **L** | **M** | **N** | **ic** | **ec** | **br** |
|---|---|---|---|---|---|---|---|---|---|---|---|---|---|---|---|---|---|
| **1** | 307,4 | 651,4 | 331,6 | 2452,3 | 2356,5 | 229,2 | 374,3 | 374,3 | 207,0 | 347,5 | 425,5 | 415,2 | 2731,9 | 393,3 | | 259,9 | 6389,3 |
| **2** | 355,0 | 354,5 | 513,9 | 1732,5 | 2204,5 | 288,3 | 445,4 | 207,6 | 341,3 | 357,7 | 214,6 | 493,7 | 2762,0 | 538,7 | 1522,5 | | 4938,9 |
| **3** | 473,3 | 1,1 | 587,5 | 2449,3 | 2774,5 | 459,9 | 382,5 | 381,5 | 420,9 | 513,5 | 297,0 | 400,9 | 2881,9 | 452,5 | | 255,2 | 5060,8 |
| **4** | 368,7 | 694,3 | 402,2 | 1764,7 | 2695,2 | 313,2 | 417,7 | 391,1 | 299,2 | 399,2 | 452,8 | 356,7 | 3650,0 | 394,2 | 1965,9 | | 5283,4 |
| **Mean** | **376,1** | **425,3** | **458,8** | **2099,7** | **2507,7** | **322,6** | **405,0** | **338,6** | **317,1** | **404,5** | **347,5** | **416,6** | **3006,5** | **444,7** | **1744,2** | **257,5** | **5418,1** |
| **SD** | **60,54** | **277,67** | **98,72** | **351,27** | **235,14** | **84,90** | **28,48** | **75,84** | **77,14** | **65,85** | **96,68** | **49,45** | **375,77** | **59,36** | **221,67** | **2,38** | **574,19** |

## Claims

1. An electrochemical DNA detection method comprising the steps of:
i) Amplifying a target DNA with a pair of amplification primers, wherein at least one of the primers is chemically linked to a label through its 5' end;
ii) Denaturing the amplified product to yield two molecules of single stranded DNA (ssDNA) of which at least one is 5'-labeled;
iii) Hybridizing a 5'-labeled ssDNA molecule of the amplified product with a complementary oligonucleotide capture probe which is attached through its 3' end to an electrode; and
iv) Determining the presence of the label associated with the hybridization product; wherein the distance between the 5'-end and the first nucleotide of the hybridization region within the ssDNA molecule that hybridises to the probe is comprised from 0 to 300 nucleotides.

2. The method according to claim 1, wherein step (iv) comprises:
a) Adding to the hybridization product a label-binding molecule to form a complex, wherein said label-binding molecule is chemically linked to an enzymatic moiety,
b) Adding to the complex resulting from step (iv) a substrate which undergoes oxidation or reduction in the presence of the enzymatic moiety; and
c) determining the oxidation or reduction of the substrate.

3. The method according to any of the claims 1-2, wherein the distance between the 5'-end and the first nucleotide of the hybridization region within the ssDNA molecule is comprised from 30 to 120, preferably from 50 to 90, and most preferably is of 83.

4. The method according to any of the claims 1 - 3, wherein the 5'-label is an hapten, preferably biotin and/or the molecule that binds the 5' label is an hapten-binding molecule, preferably streptavidin, avidin or neutravidin, and/or the enzymatic moiety is an oxido-reductase, preferably HRP or polyHRP, and/or the substrate is an electron donor, preferably 3,3',5,5'-tetramethylbenzidine (TMB).

5. The method according to any of the claims 1 - 4, that is for detection and/or typing of *Human Papillomavirus.*

6. Use of means for electrochemical DNA detection as defined in any of the claims 1-5, wherein the means comprise:
i) A pair of primers to amplify the DNA target, wherein at least one of the primers is chemically linked to a label through its 5' end;
ii) An oligonucleotide capture probe complementary to the DNA target sequence, wherein the oligonucleotide capture probe is attached through its 3' end to an electrode; and
iii) Means to detect the label;
Wherein when in use the distance between the 5'-end and the first nucleotide of the hybridization region within the ssDNA molecule that hybridises to the probe is comprised from 0 to 300 nucleotides.

7. Cartridge device for electrochemical DNA detection including a microfluidic circuit comprising:
a microfluidic amplification chamber operable to cause amplification of a target DNA with a pair of amplification primers, wherein at least one of the primers is chemically linked to a label through its 5' end, and to cause denaturation of the amplified product to yield two molecules of single stranded DNA (ssDNA) of which at least one is 5'-labeled; and
a microfluidic sensor chamber comprising one or more electrodes and one or more complementary oligonucleotide capture probes attached through their 3' end to the one or more electrodes, and being operable to cause hybridization of the at least one 5'-labeled ssDNA molecule of the amplified product with the one or more complementary oligonucleotide capture probes, the distance upon hybridization between the 5'-end and the first nucleotide of the hybridization region within the ssDNA molecule that hybridises to the probe being of from 0 to 300 nucleotides;
wherein
the microfluidic sensor chamber is further operable to cause determination of the presence of the label associated with the hybridization product.

8. Cartridge device as defined in claim 7, further comprising a container containing an amplification buffer with the pair of amplification primers and in fluid communication with the microfluidic circuit, so that the amplification buffer is transferrable to the microfluidic amplification chamber.

9. Cartridge device as defined in any of the claims 7 or 8, wherein the microfluidic sensor chamber is operable to cause determination of the presence of the label associated with the hybridization product in such a way that
addition to the hybridization product of a label-binding molecule to form a complex is caused, wherein said label-binding molecule is chemically linked to an enzymatic moiety;
addition to the formed complex of a substrate which undergoes oxidation or reduction in the presence of the enzymatic moiety is caused; and
determination of the oxidation or reduction of the substrate is caused.

10. Cartridge device as defined in claim 9, further comprising a container containing a buffer with the label-binding molecule chemically linked to the enzymatic moiety; and
a container containing a buffer with the substrate which undergoes oxidation or reduction in the presence of the enzymatic moiety; wherein
said containers are in fluid communication with the microfluidic circuit, so that said buffers are transferrable to the microfluidic sensor chamber.

11. Method of controlling an operator device for operating a cartridge device as defined in any of the claims 7 to 10, which is associable to the operator device, the method comprising:
providing, by a controller of the operator device, control signal to a first heater unit of the operator device for operating the microfluidic amplification chamber of the cartridge device by providing first heating cycles to the microfluidic amplification chamber, so that amplification and denaturation occur in the microfluidic amplification chamber;
providing, by the controller, control signal to a second heater unit of the operator device for operating the microfluidic sensor chamber of the cartridge device by providing second heating cycles to the microfluidic sensor chamber, so that hybridization occurs in the microfluidic sensor chamber; and
providing, by the controller, control signal to a detection unit of the operator device for operating the microfluidic sensor chamber so that determination of the presence of the label associated with the hybridization product occurs.

12. Operator device for operating a cartridge device which is associable to the operator device, wherein
the operator device comprises a first heater unit, a second heater unit, a detection unit, and
a controller comprising electronic means for performing a method as defined in claim 11 for controlling the operator device.

13. Operator device as defined in the claim 12, wherein the detection unit comprises a reading contactor for contacting the electrodes and obtaining one or more readings therefrom.

14. Operator device for operating a cartridge device which is associable to the operator device, wherein
the operator device comprises a first heater unit, a second heater unit, a detection unit, and
a controller comprising a memory and a processor, embodying instructions stored in the memory and executable by the processor, the instructions comprising functionality to execute a method as defined in claim 11 for controlling the operator device.

15. Computer program product comprising program instructions for causing a controller of an operator device to perform a method as defined in claim 11 of controlling an operator device for operating a cartridge device.
